# EUROPEAN PATENT APPLICATION

(11) **EP 4 847 077 A1**
(43) Date of publication of application: **02.12.2026**
(21) Application number: 25178886.5
(22) Date of filing: 26.05.2025
(51) Int. Cl.: A61N 1/04, A61N 1/39

(54) **DEFIBRILLATOR PADDLE WITH ADJUSTABLE CONTACT AREA**

(71) Applicant: Everest Acquisition Entity, LLC, Wilmington, DE 19805 (US)
(72) Inventor: XIE, Wencheng, 5656 AG Eindhoven (NL); WANG, Aihua, 5656 AG Eindhoven (NL); FU, Hongping, 5656 AG Eindhoven (NL)
(74) Representative: Weilnau, Carsten

(57) **Abstract**

A defibrillator paddle comprising a first electrode portion and one or more second electrode portions. The one or more second electrode portions are movable with respective to the first electrode portion, said movement being driven by a transmission mechanism of the defibrillator paddle. Through moving the one or more second electrode portions, the first electrode portion may be combined with different second electrode portions to form different active electrodes of the defibrillator paddle. Each active electrode is configured for defibrillation of a subject and is associated with a respective defibrillation mode of the defibrillator paddle.

## Description

### FIELD OF THE INVENTION

The present invention relates to the field of defibrillators, and in particular to defibrillator paddles comprising adjustable contact areas.

### BACKGROUND OF THE INVENTION

Defibrillators are commonly used to treat patients suffering from cardiac arrest and/or heart arrythmia. The defibrillator generates a short burst of electrical current which is transferred to the patient through a pair of defibrillator paddles pressed against a part of the patient's body. This provides an electrical shock to the patient's heart, resetting the heart's cardiac cycle.

Defibrillator paddles typically comprise an electrode or electrode plate which is pressed against the patient's body to transfer the electrical shock. The lateral size of the electrode is preferably tailored according to the size of the patient to ensure full contact between the electrode surface and the patient's body. In particular, child or infant patients require much smaller electrode plates than adult patients.

Some defibrillator paddles comprise removeable adult electrodes that expose child electrodes, allowing the defibrillator paddles to be used for defibrillation of different sized patients. However, this configuration can result in increased wear to the child electrode due to friction with the adult electrode. Additionally, the adult electrode can become lost due to not forming an integral part of the defibrillator paddle. There is thus on ongoing desire for alternative defibrillator paddles capable of defibrillating patients of different type and/or size.

### SUMMARY OF THE INVENTION

The invention is defined by the claims.

According to examples in accordance with an aspect of the invention, there is provided a defibrillator paddle comprising: a plurality of electrode portions, each electrode portion comprising a first side; a second side, opposite to the first side; and a shock surface on the first side of the electrode portion, wherein the plurality of electrode portions comprises: a first electrode portion; and one or more second electrode portions configured to be moveable with respect to the first electrode portion; and a transmission mechanism configured to drive movement of the one or more second electrode portions relative to the first electrode portion.

The defibrillator paddle is switchable between at least two defibrillation modes for defibrillation of a subject, wherein each of the at least two defibrillation modes is associated with: a respective positional arrangement of the one or more second electrode portions relative to the first electrode portion; and a respective subset of the first electrode portion and the one or more second electrode portions forming an active electrode of the defibrillator paddle for defibrillation of the subject, wherein the respective subset comprises either i) only the first electrode portion or ii) the first electrode portion and at least one of the one or more second electrode portions. The transmission mechanism is configured to switch the defibrillator paddle between the at least two defibrillation modes by driving movement of at least one of the one or more second electrode portions.

The present disclosure provides a defibrillator paddle that can switch between at least two different defibrillation modes, each utilizing a different electrode portion configuration for defibrillation of a subject. In particular, each defibrillation mode utilizes a distinct set of electrode portions to form an active electrode of the defibrillator paddle, the active electrode comprising electrode portions intended to be used for defibrillation of the subject (i.e., come into contact with the subject when providing defibrillation).

The defibrillator paddle comprises a transmission mechanism for switching the defibrillator paddle between different defibrillation modes. Specifically, the transmission mechanism drives (i.e., controls) movement of different electrode portions of the defibrillator paddle (relative to other electrode portions) in order to arrange the electrode portions into a respective configuration for each defibrillation mode (at least some of the arranged electrode portions forming the active electrode for the defibrillation mode).

The proposed approach enables the configuration of the active electrode for the defibrillator paddle to be changed (i.e., through switching between different defibrillation modes) thereby allowing the active electrode to be preferably modified according to a target subject.

In some examples, in each of the at least two defibrillation modes of the defibrillator paddle, the shock surface of each electrode portion forming the active electrode for the defibrillation mode lies in a predefined plane for said defibrillation mode. This may be a same predefined plane for each shock surface or a respective (e.g., different) predefined plane for each shock surface.

In some examples, each active electrode for the at least two defibrillation modes of the defibrillator paddle is associated with a shock surface area being the sum of the area of the shock surface of each electrode portion forming the active electrode; and each active electrode for the at least two defibrillation modes of the defibrillator paddle is associated with a different shock surface area. Each defibrillation mode thereby provides a different sized contact area between the defibrillator paddle and a subject. Accordingly, the defibrillator paddle may be switched between different modes dependent on a size of the subject and/or a preferred (active) electrode area.

In some examples, in each of the at least two defibrillation modes of the defibrillator paddle, each electrode portion forming the active electrode for the defibrillation mode is in physical contact with at least one other electrode portion forming said active electrode. For example, the shock surfaces of each electrode portion forming the active electrode may form a continuous/contiguous shock surface of the active electrode.

In some examples, each electrode portion comprises a flexible material. This allows each electrode portion to be (at least partially and/or temporarily) deformed, improving the maneuverability of each electrode portion, in particular the maneuverability of each second electrode portion relative to the first electrode portion.

In some examples, the second side of each electrode portion is covered in a non-conductive support layer. This enhances the rigidness of each electrode portion (e.g., when the electrode portion is flexible) and prevents transfer of an electrical shock through the second side of the electrode portion.

In some examples, in each of the at least two defibrillation modes of the defibrillator paddle, any second electrode portion not forming the active electrode for the defibrillation mode is arranged in a region facing the second side of each electrode portion forming said active electrode. Each second electrode portion not forming the active electrode is thus positioned behind the active electrode, ensuring said second electrode portions are out of the way during defibrillation.

In some examples, the defibrillator paddle is configured to receive an electrical shock from an electrical shock system; and, in each of the at least two defibrillation modes of the defibrillator paddle, the electrical shock is provided to at least each electrode portion forming the active electrode for the defibrillation mode. Thus, each electrode portion forming the active electrode is capable of transferring the electrical shock to a subject.

In some examples, the electrical shock is provided to the first electrode portion; and, in each of the at least two defibrillation modes of the defibrillator paddle, each second electrode portion forming the active electrode for the defibrillation mode is configured to form an electrical connection with the first electrode portion. Each second electrode portion forming the active electrode thus receives the electrical shock via the first electrode portion.

In some examples, any second electrode portion not forming part of the active electrode is electrically isolated from the first electrode portion. Accordingly, each second electrode portion may only receive an electrical shock when it forms part of the active electrode.

In some examples, the defibrillator paddle further comprises an insulating cover arranged in a region facing the second side of each electrode portion forming the active electrode for any defibrillation mode of the defibrillator paddle, wherein, in each of the at least two defibrillation modes of the defibrillator paddle, a projection of the active electrode for the defibrillation mode onto the insulating cover is entirely contained within bounds of the insulating cover. The insulating cover forms a physical barrier that separates the active electrode (at one side of the insulating cover) from anything at another side of the insulating cover. Accordingly, the insulating cover may be used as a guard, e.g., to protect a user's hand when operating the defibrillator paddle.

In some examples, the bounds of the insulating cover are configured to change responsive to the defibrillation mode of the defibrillator paddle. In particular, the bounds of the insulating cover may be reduced in size when the projection of the active electrode onto the insulating cover is smaller (and vice versa when the projection is bigger). In other words, the insulating cover may be scaled responsive to the size of the active electrode. This reduces an amount of redundant insulating cover area for each defibrillation mode of the defibrillator paddle.

In some examples, the defibrillator paddle further comprises a user-controlled switch switchable between at least two switching states, each switching state being associated with a respective defibrillation mode of the at least two defibrillation modes of the defibrillator paddle; and the transmission mechanism is configured to switch the defibrillator paddle to any one of the at least two defibrillation modes in response to the user-controlled switch being in the switching state associated with said defibrillation mode. The defibrillation mode in which the defibrillator paddle operates is thus dependent on the switching state of the user-controlled switch, e.g., as set by a user.

There is further provided a defibrillator comprising: an electrical shock system; a processing system configured to control the electrical shock system; and at least one defibrillator paddle according to the previous disclosure, wherein the at least one defibrillator paddle is configured to receive an electrical shock from the electrical shock system.

In some examples, the processing system is configured to control at least an energy of any electrical shock provided by the electrical shock system to the at least one defibrillator paddle responsive to an indication of the at least one defibrillator paddle operating in any one of the at least two defibrillation modes.

In some examples, the defibrillator further comprises, for each defibrillator paddle, a paddle tray configured to receive the defibrillator paddle in any defibrillation mode of the defibrillator paddle.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a proposed defibrillator paddle;
Fig. 2 illustrates different defibrillation modes of the proposed defibrillator paddle;
Fig. 3 illustrates a transmission mechanism of the proposed defibrillator paddle;
Fig. 4 illustrates a further proposed defibrillator paddle;
Fig. 5 illustrates different defibrillation modes of the further proposed defibrillator paddle; and
Fig. 6 illustrates a proposed paddle tray.

### DETAILED DESCRIPTION OF THE EMBODIMENTS

The invention will be described with reference to the Figures.

It should be understood that the detailed description and specific examples, while indicating exemplary embodiments of the apparatus, systems, and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a defibrillator paddle comprising a first electrode portion and one or more second electrode portions. The one or more second electrode portions are movable with respective to the first electrode portion, said movement being driven by a transmission mechanism of the defibrillator paddle. Through moving the one or more second electrode portions, the first electrode portion may be combined with different second electrode portions to form different active electrodes of the defibrillator paddle. Each active electrode is configured for defibrillation of a subject and is associated with a respective defibrillation mode of the defibrillator paddle.

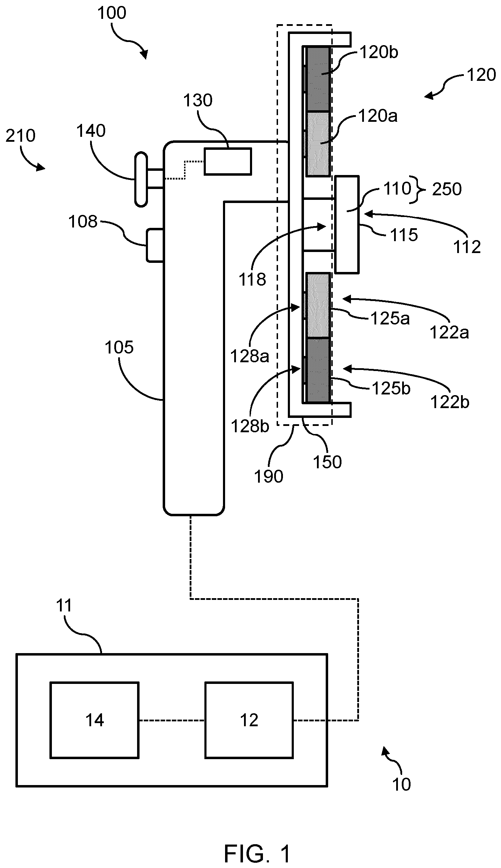

Fig. 1 illustrates a defibrillator 10 comprising a proposed defibrillator paddle 100 according to an embodiment of the invention. The defibrillator 10 is adapted for defibrillation of a subject using an electrical shock provided via the defibrillator paddle 100. The defibrillator 10 may also comprise one or more further defibrillator paddles in addition to the proposed defibrillator paddle 100 (e.g., a second proposed defibrillator paddle). In particular, a pair of defibrillator paddles may be used in combination to provide the electrical shock to the subject, i.e., through forming an electrical path through the subject between the two defibrillator paddles.

The defibrillator 10 further comprises an electrical shock system 12 configured to provide (when prompted) the electrical shock to the defibrillator paddle 100 for defibrillation of the subject. In particular, when the electrical shock system 12 (or the defibrillator 10 as a whole) is in an 'ON' state, i.e., receiving electrical power from a mains supply or internal battery, the electrical shock system 12 may store electrical energy, e.g., in a capacitor. Subsequently, following an activation indicator (e.g., a user input), the electrical shock system 12 may release said electrical energy as an electrical shock and provide it to the defibrillator paddle 100. Said activation indicator may be provided (e.g., by a user) following the defibrillator paddle 100 being ready for defibrillation, e.g., correctly positioned on the subject.

The defibrillator paddle 100 may only receive the electrical shock when (electrically) connected to the electrical shock system 12, e.g., via an electrical cable/wire. In some examples, the defibrillator paddle 100 is disconnectable/detachable from the electrical shock system 12, e.g., through disconnecting the electrical cable/wire from the electrical shock system 12 and/or the defibrillator paddle 100.

The defibrillator 10 further comprises a processing system 14 configured to control the electrical shock system 12. For example, the processing system 14 may control provision of the electrical shock to the defibrillator paddle 100 from the electrical shock system 12 (e.g., in response to receiving an activation indicator) and/or control an energy of said electrical shock.

The electrical shock system 12 and the processing system 14 may form part of (e.g., be integrated into) a defibrillator monitor 11. The defibrillator monitor 11 may comprise one or more controls, interfaces, or displays to allow a user to interact with, modify settings of, and/or perceive information regarding the electrical shock system 12 and/or the processing system 14.

The defibrillator paddle 100 comprises a handle 105 configured to be held by a user when operating the defibrillator paddle 100. In this context, operation of the defibrillator paddle 100 refers to use of the defibrillator paddle 100 for application of the electrical shock to the subject. The user may grip the handle 105 to maneuver the defibrillator paddle 100 and bring a part of the defibrillator paddle 100 into contact with the subject to apply the electrical shock.

The defibrillator paddle 100 also comprises a button 108 arranged on the handle 105. When activated (e.g., pressed) by a user, the button 108 enables transmission of the electrical shock to the subject. For example, activation of the button 108 may trigger closing of a switch to allow transmission of the electrical shock through the defibrillator paddle 100. In some examples, activation of the button 108 may (also) generate an activation indicator (e.g., carried by or embodied as an electrical signal) that is provided to the processing system 14 (which in response causes the electrical shock system 12 to release the electrical shock). The button 108 therefore allows a user to control when the subject is defibrillated.

Other (mechanical) actuators or activating elements to be used in place of the button 108 will be readily known to the skilled person. For example, the button 108 may be replaced by a switch, dial, slider, touchscreen, or other user-interactable element.

In accordance with the present disclosure, the defibrillator paddle 100 comprises a plurality of electrode portions adaptable for provision of the electrical shock to the subject. In particular, a relative positional arrangement of the plurality of electrode portions may be modified in order to preferably adapt the defibrillator paddle 100 for defibrillation of the subject based on characteristics of the subject, e.g., size.

The defibrillator paddle 100 comprises a first electrode portion 110 and one or more second electrode portions 120. Specifically, in the present example, the defibrillator paddle 100 comprises a first second electrode portion 120a and a second second electrode portion 120b, i.e., two second electrode portions. As will become evident, the defibrillator paddle 100 may comprise any number of second electrode portions greater than or equal to one, e.g., one second electrode portion, three second electrode portions, five second electrode portions, ten second electrode portions, etc.

Each electrode portion of the defibrillator paddle 100 is configured for transmitting/delivering the electrical shock to the subject during defibrillation. Particularly, when provided with (a portion of) the electrical shock from the electrical shock system 12, each electrode portion is configured to transfer the electrical shock to the subject when held against the subject.

More specifically, each electrode portion of the defibrillator paddle 100 comprises a shock surface, being the surface of the electrode portion intended to come into contact with the subject when said electrode portion is used for defibrillation. Accordingly, during defibrillation, the electrical shock is transferred from the defibrillator paddle 100 to the subject via the shock surface of each electrode portion being used.

Each shock surface may comprise (or be coated in) a conductive material, e.g., a metal or metal alloy. More generally, each electrode portion may comprise one or more conductive portions for facilitating transfer of the electrical shock to the subject.

Each electrode portion may be embodied as a plate or block with the shock surface arranged on a defined side/surface of the electrode portion. Additionally, the shock surface for each electrode portion may define a directionality of the electrode portion, with the shock surface arranged on a front (i.e., first) side of the electrode portion. Thus, a back (i.e., second) side of the electrode portion corresponds to the side/surface of the electrode portion opposite the shock surface.

To be precise, in the present example, the first electrode portion 110 comprises a first shock surface 115 arranged on a first side 112 (of the first electrode portion 100) opposite a second side 118, the first second electrode portion 120a comprises a first second shock surface 125a arranged on a first side 122a (of the first second electrode portion 120a) opposite a second side 128a, and the second second electrode portion 120b comprises a second second shock surface 125b arranged on a first side 122b (of the second second electrode portion 120b) opposite a second side 128b.

Each of the one or more second electrode portions 120 is configured to be moveable, e.g., translatable, and/or rotatable, with respect to the first electrode portion 110. Thus, the positional arrangement of the one or more second electrode portions 120 relative to the first electrode portion 110 may be changed.

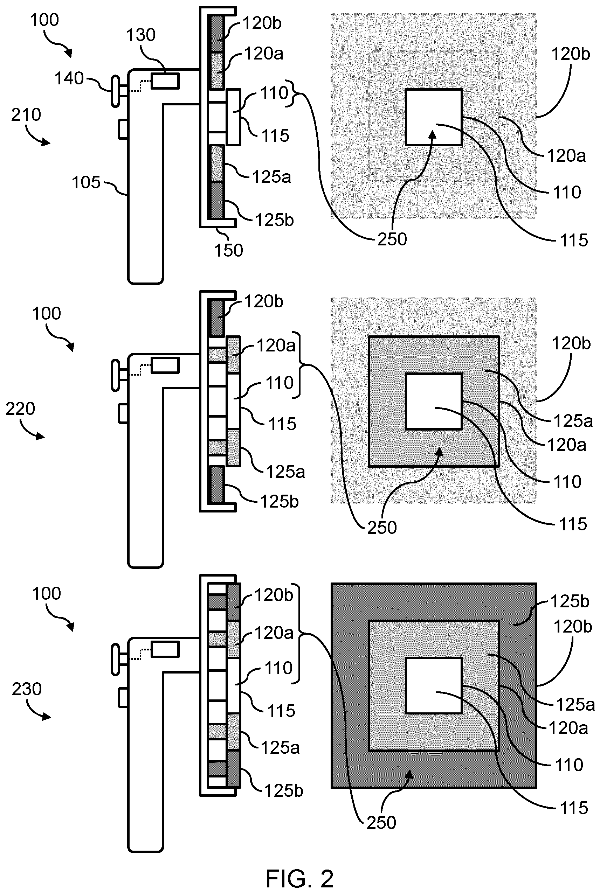

For instance, in the present example (and as shown in Fig. 2) each of the one or more second electrode portions 120 is translatable along a linear axis such that their alignment with the first electrode portion 110 may be changed. In particular, each of the one or more second electrode portions 120 is translatable between a retracted state (as depicted in Fig. 1) and an extended state in which the shock surface of the second electrode portion aligns with the first shock surface 115.

In the present example, the position and orientation of the first electrode portion 110 on the defibrillator paddle 100 is fixed, i.e., non-moveable. In other words, only the one or more second electrode portions 120 are configured to be moveable. In other examples, the first electrode portion 110 may also be moveable, e.g., with respect to a remainder of the defibrillator paddle 100.

Movement of the one or more second electrode portions 120 is driven by a transmission mechanism 130 of the defibrillator paddle 100. In particular, when activated, the transmission mechanism 130 may drive movement of one or more moveable parts (of the transmission mechanism 130) which act on the one or more second electrode portions 120, causing movement thereof. Such movement may be driven by an electrical, mechanical, hydraulic, or pneumatic mechanism, or a combination thereof, where said mechanism utilizes one or more gears, motors, actuators, etc., as will be familiar to the skilled person.

Activation of the transmission mechanism 130 may be controlled via a user-controlled switch 140 of the defibrillator paddle 100. The user-controlled switch 140 is switchable between (at least two) different switching states, e.g., as set by a user. Each switching state causes activation of the transmission mechanism 130 and subsequent movement of at least some of the one or more second electrode portion 120. In particular, each switching state may be associated with a respective positional arrangement of the one or more second electrode portions 120 relative to the first electrode portion 110. Thus, each switching state may activate the transmission mechanism 130 in such a way as to produce the respective positional arrangement for said switching state, e.g., move the second electrode portions whose positions do not conform with the respective positional arrangement, said movement resulting in said second electrode portions conforming with the respective positional arrangement.

In some examples, the user-controlled switch 140 comprises an electronic switching element (e.g., a switch, slider, button(s), touchscreen, etc.) that produces an electrical signal indicative of the switching state of the user-controlled switch 140. The transmission mechanism 130 may process said electrical signal and drive movement of the one or more second electrode portions 120 accordingly. Alternatively, said electrical signal may innately trigger activation of the transmission mechanism 130 from providing an electrical voltage/current to electrical elements of the transmission mechanism 130.

In some examples, the user-controlled switch 140 comprises a mechanical switching element such as a rotating dial, gear, knob, or lever. Accordingly, mechanical (e.g., rotational) movement of the user-controlled switch 140 drives movement of the one or more second electrode portions 120 via the transmission mechanism 130, e.g., through driving one or more gears of the transmission 130. In other words, mechanical movement/switching of the user-controlled switch 140 is translated to movement of the one or more second electrode portions 120 by the transmission mechanism 130.

The transmission mechanism 130 (and the user-controlled switch 140) thus allow a user to indirectly move the one or more second electrode portions 120, i.e., through activating/driving the transmission mechanism 130. In other words, the one or more second electrode portions 120 may be moved without a user having to physically interact with them. This reduces a risk of a user damaging the one or more second electrode portions 120 (or a movement mechanism thereof), e.g., through improper handling of, or applying excessive force to, the one or more second electrode portions 120.

The present disclosure recognizes that some positional arrangements of the one or more second electrode portions 120 relative to the first electrode portion 110 may provide a preferable arrangement for defibrillation of a subject. Particularly, in some positional arrangements, certain electrode portions may be used in combination to provide an effective, operative, or "active" electrode with particular characteristics or properties, e.g., a particular size and/or shape. Said characteristics/properties of the active electrode may be preferable for defibrillating particular types of subjects, e.g., subjects of a particular size and/or age. Accordingly, in such positional arrangements, only electrode portions forming the active electrode may be used (or be intended to be used) for defibrillating a subject.

The present disclosure proposes configuring the defibrillator paddle 100 to be operable in different defibrillation modes, each associated with (or assigned to) a respective positional arrangement of the one or more second electrode portions 120 relative to the first electrode portion 110. More particularly, each defibrillation mode of the defibrillator paddle 100 is associated with a respective subset of the first electrode portion 110 and the one or more second electrode portions 120 (i.e., a respective set of electrode portions chosen from the first electrode portion 110 and the one or more second electrode portions 120) that form an active electrode when arranged according to the respective positional arrangement for the defibrillation mode.

For the sake of conciseness, a defibrillation mode of a defibrillator paddle may simply be referred to as a defibrillation mode. Thus, any (implicit) reference to something being in, or switched to, a defibrillation mode will be understood to mean a defibrillator paddle being in, or switched to, the defibrillation mode.

The respective subset for each defibrillation mode comprises (at least) the first electrode portion 110 and, for some defibrillation modes, at least one of the one or more second electrode portions 120. The active electrodes for each defibrillation mode thus differ in terms of which second electrode portion(s) (if any) is/are used in combination with the first electrode portion 110 to form the active electrode.

Furthermore, the defibrillator paddle 100 is switchable between different defibrillation modes by means of the transmission mechanism 130. Specifically, the transmission mechanism 130 is configured to switch the defibrillator paddle 100 between different defibrillation modes by driving movement of (at least one of) the one or more second electrode portions 120, thereby modifying the positional arrangement of the electrode portions from a respective positional arrangement for one defibrillation mode to a respective positional arrangement for another defibrillation mode.

It will be appreciated that the simplest form of defibrillator paddle (according to the present disclosure) may comprise only a single second electrode portion. In such examples, only two different active electrodes may be formed: one comprising only the first electrode portion; and another comprising the first electrode portion and the second electrode portion. Accordingly, the defibrillator paddle may only operate in two different defibrillation modes, each associated with a different active electrode composition. Any herein proposed defibrillator paddle is therefore switchable between at least two (different) defibrillation modes, e.g., dependent on the number of second electrode portions that the defibrillator paddle comprises.

In Fig. 1, the defibrillator paddle 100 is depicted in a first defibrillation mode 210. In this defibrillator mode, each of the one or more second electrode portions 120 is in a retracted state. Specifically, each of the one or more second electrode portions 120 is arranged behind the first electrode portion (or at least the shock surface of the second electrode portion is arranged further back than the first shock surface 115) corresponding to being in the retracted state.

Accordingly, due to being the forward-most electrode portion, the first electrode portion 110 is preferably positioned (over each of the one or more second electrode portions 120) for defibrillating the subject. In particular, when the first shock surface 115 is brought into contact with the subject, each of the one or more second electrode portions 120 may be separated from the subject by a gap, thus preventing or hindering the second electrode portion from providing the electrical shock to the subject. In other words, only the first electrode portion 110 may be used for defibrillating the subject. The first electrode portion 110 thus forms an active electrode 250 of the defibrillator paddle 100 when in the first defibrillation mode 210.

The defibrillator paddle 100 may operate in the first defibrillation mode 210 responsive to the user-controlled switch 140 being in (or switched to) a first switching state. Particularly, in response to said switching state, the transmission mechanism 130 may drive movement of the one or more second electrode portions 120 so that they are in the retracted state, thus providing the respective positional arrangement associated with the first defibrillation mode 210.

A region 190 may be defined behind the active electrode 250. More specifically, the region 190 (or a side/surface thereof) faces the second side of each electrode portion forming active electrode 250. For instance, in the first defibrillator mode 210, the region 190 faces the second side 118 of the first electrode portion. The region 190 is therefore arranged at a side of the active electrode 250 opposite to a side comprising the shock surface of each electrode portion forming the active electrode 250.

It will be appreciated here that a side/surface may only "face" another side/surface when both sides/surfaces are facing or pointing towards each other.

In the present example, each of the one or more second electrode portions 120 is arranged in the region 190. More generally, in each defibrillation mode of the defibrillator paddle 100, any second electrode portion not forming the active electrode 250 (for the defibrillation mode) may be arranged within the region 190. In this way, each second electrode portion not forming the active electrode 250 is positioned behind the active electrode 250, thus preventing (or at least reducing a risk of) said second electrode portions from coming into contact with a subject. Particularly, when the active electrode 250 is brought into contact with the subject, the active electrode 250 may form a physical barrier between the subject and each second electrode portion not forming the active electrode 250, or at least create a gap/space between the subject and said second electrode portions, such that said second electrode portions are not in contact with the subject. Additionally, arranging each second electrode portion not forming the active electrode 250 in this way also prevents (or at least reduces a risk of) said second electrode portions blocking or hindering the active electrode 250 from coming into contact with the subject.

Fig. 2 illustrates further defibrillation modes of the defibrillator paddle 100, i.e., a second defibrillation mode 220 and a third defibrillation mode 230. Each defibrillation mode is associated with a different (i.e., respective) positional arrangement of the first second electrode portion 120a and the second second electrode portion 120b relative to the first electrode portion 110, corresponding to whether each second electrode portion is in a retracted or extended state. Furthermore, each defibrillation mode is associated with a respective active electrode 250 formed from a respective combination of the first electrode portion 110, the first second electrode portion 120a, and the second second electrode portion 120b.

In the second defibrillation mode 220, the first second electrode portion 120a is extended and the second electrode portion 120b is retracted. Accordingly, both the first electrode portion 110 and the first second electrode portion 120a form the active electrode 250 of the defibrillator paddle 100. In particular, in the second defibrillation mode 220, the first second electrode portion 120a contacts the first electrode portion 110 and their shock surfaces form a (semi-) contiguous shock surface of the active electrode 250.

The defibrillator paddle 100 may be switched to the second defibrillation mode 220 responsive to the user-controlled switch 140 being switched to a second switching state. Particularly, in response to said switching state, the transmission mechanism 130 may drive movement of the first second electrode portion 120a into the extended state and the second second electrode portion 120b into the retracted state (e.g., if they weren't already in said states), thus providing the respective positional arrangement associated with the second defibrillation mode 220.

In the third defibrillation mode 230, both the first second electrode portion 120a and the second electrode portion 120b are extended. Accordingly, the first electrode portion 110, the first second electrode portion 120a, and the second second electrode portion 120b together form the active electrode 250 of the defibrillator paddle 100. In particular, in the third defibrillation mode 230, the first second electrode portion 220a contacts the first electrode portion 110, the second second electrode portion 220b contacts the first second electrode portion 220a, and their shock surfaces form a (semi-) contiguous shock surface of the active electrode 250.

The defibrillator paddle 100 may be switched to the third defibrillation mode 230 responsive to the user-controlled switch 140 being switched to a third switching state. Particularly, in response to said switching state, the transmission mechanism 130 may drive movement of the first second electrode portion 120a and the second second electrode portion 120b into the extended state (e.g., if they weren't already in said state), thus providing the respective positional arrangement associated with the third defibrillation mode 230.

In the present example, the shock surfaces of each electrode portion forming the active electrode 250 all lie in a same predefined plane, as defined by the plane in which the first shock surface 115 lies. The active electrode 250 in each defibrillation mode thus effectively comprises a flat plane/plate.

In other examples, the shock surfaces of each electrode portion forming the active electrode may lie in different (predefined) planes, e.g., intersecting planes. In such examples, the active electrode may comprise an angled or (approximately) curved surface. This may provide a better shape/surface (of the active electrode) for fitting the active electrode around a part of the subject, e.g., so that better or increased contact is made between the active electrode and the subject.

In the present example, each active electrode 250 for the different defibrillation modes is of a different (lateral) size. This size may be quantified in terms of a shock surface area for each active electrode 250, being the total (i.e., summed) area of all the shock surfaces forming the active electrode 250. Accordingly, each active electrode 250 for the different defibrillation modes has a different shock surface area. For instance, the shock surface area (for the active electrode 250) is smaller in the first defibrillation mode 210 compared to the second defibrillation mode 220, and the shock surface area is smaller in the second defibrillation mode 220 compared to the third defibrillation mode 230. This advantageously allows the shock surface area (and thus size) of the active electrode 250 to be tuned, e.g., depending on the size and/or age of the subject. Particularly, a smaller active electrode is preferably used when defibrillating smaller subjects such as infants and children. Thus, the defibrillator paddle 100 may be switched to the first defibrillation mode 210 or the second defibrillation mode 220 to obtain an active electrode 250 of reduced size.

In addition to a reduced active electrode size, defibrillation of infant and child subjects preferably uses electrical shocks of a lower (electrical) energy compared to those used on adult subjects. Accordingly, when each defibrillation mode is intended to be used for defibrillating a subject of a particular age and/or size, each defibrillation mode may also utilize (or otherwise control) an electrical shock of a particular (e.g., predetermined) energy. In other words, the energy of an electrical shock received by the defibrillator paddle 100 (e.g., from the electrical shock system 12 of Fig. 1) may be responsive to the defibrillation mode of the defibrillator paddle 100.

In some examples, the transmission mechanism 130 (or some other element of the defibrillator paddle 100) is configured to generate an indicator (e.g., an electrical signal) indicative of the defibrillation mode in which the defibrillator paddle 100 is operating. The transmission mechanism 130 may generate the indicator while the defibrillator paddle 100 is operating in the defibrillation mode (e.g., continuously, or periodically), or generate the indicator on switching the defibrillator paddle 100 to the defibrillation mode. The transmission mechanism may provide the indicator to the processing system 14 of Fig. 1, e.g., via an electrical cable connected to the defibrillator monitor 11. Responsive to said indicator, the processing system 14 may control the energy of any electrical shock provided by the electrical shock system, e.g., to be equal to, or no greater than, a predetermined energy associated with defibrillation mode of the defibrillator paddle 100 (as determined from the indicator).

In other examples, an indication of the defibrillation mode in which the defibrillator paddle 100 is operating may be provided to the processing system 14 by a user, e.g., via a user-interface of the defibrillator monitor 11.

In some examples, the defibrillator paddle 100 further comprises an insulating cover 150. For example, in Fig. 1, the insulating cover 150 is depicted as lying within the region 190. In general, however, the insulating cover may be arranged within any region facing the second side of each electrode portion forming the active electrode 250 (i.e., arranged behind the active electrode 250).

The insulating cover 150 comprises an insulating material (e.g., plastic) and forms a protective barrier between the active electrode 250 and other parts of the defibrillator paddle 100. In particular, the insulating cover 150 may form a barrier between the active electrode 250 and the handle 105 to protect a user's hand when holding the defibrillator paddle 100, e.g., to reduce a risk of the user being electrocuted by the active electrode 250 when operating the defibrillator paddle 100. In each defibrillation mode, a projection of the active electrode 250 for the defibrillation mode onto the insulating cover 150 may be entirely contained within bounds (e.g., edges) of the insulating cover 150. In this way, a lateral extent of the insulating cover 150 is greater than that of the active electrode 250, providing greater protection from the active electrode 250.

The insulating cover 150 may also form a barrier between any second electrode portions not forming the active electrode 250 and other parts of the defibrillator paddle 100. Accordingly, each second electrode portion not forming the active electrode 250 may be arranged between the insulating cover 150 and the active electrode 250.

As previously mentioned, the electrical shock received by the defibrillator paddle 100 (e.g., from the electrical shock system 12 of Fig. 1) is provided to (at least some of) the electrode portions of the defibrillator paddle 100 for transfer to the subject (e.g., via electrode portions forming the active electrode 250). In some examples, the electrical shock is only provided to some (i.e., not all) of the electrode portions dependent on the defibrillation mode of the defibrillator paddle 100 (and thus positional arrangement of the electrode portions). For instance, the electrical shock may only be provided to electrode portions forming the active electrode 250 for the defibrillation mode. Thus, the electrical shock may only be transferred to the subject via electrode portions intended to be used for defibrillation.

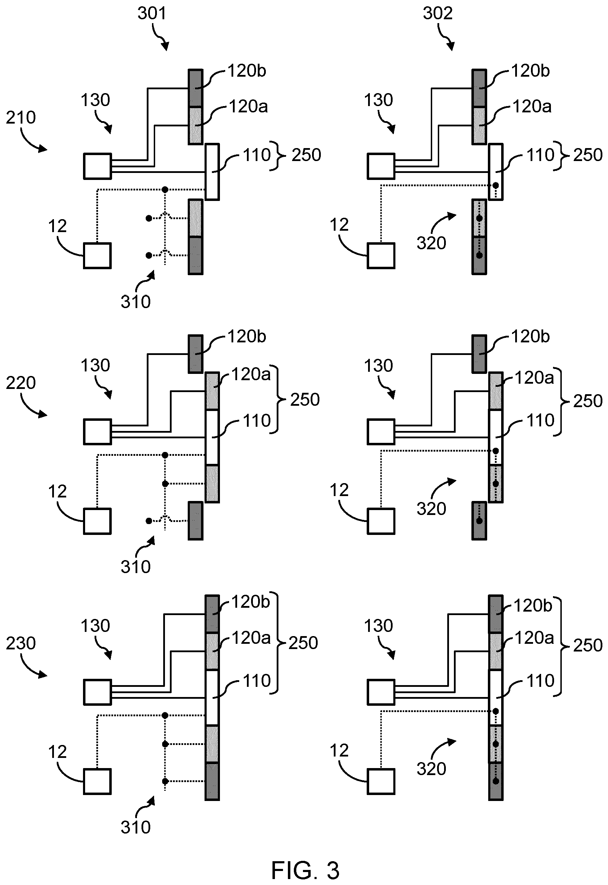

Fig. 3 illustrates a first approach 301 and a second approach 302 by which provision of the electrical shock from the electrical shock system 12 to different electrode portions can be controlled dependent on the defibrillation mode of the defibrillator paddle 100.

According to the first approach 301, the defibrillator paddle 100 comprises first electrical circuitry 310 that allows each electrode portion to electrically couple to the electrical shock system 12. Specifically, the first electrical circuitry 310 comprises a plurality of signal lines, each connected to a respective electrode portion and allowing said electrode portion to couple to the electrical shock system 12.

In this example, each electrode portion is electrically isolated from every other electrode portion, or at least not allowed to come into electrical contact with any other electrode portion, such that a direct electrical connection cannot be made between electrode portions. Thus, each electrode portion may only receive the electrical shock via their corresponding signal line of the first electrical circuitry 310, e.g., and not via another electrode portion.

As the first electrode portion 110 is always used to form the active electrode 250, the signal line to the first electrode portion 110 always (i.e., independent of the defibrillation mode) forms a complete connection to the electrical shock system 12. Thus, the first electrode portion 110 may always receive the electrical shock from the electrical shock system 12.

Conversely, the signal line to each second electrode portion is configured to only form a complete connection to the electrical shock system 12 when the second electrode portion forms part of the active electrode 250. Specifically, when each electrode portion is moved by the transmission mechanism 130 to form part of the active electrode 250, the signal line to the second electrode portion also moves resulting in the signal line forming a complete connection to the electrical shock system 12. Put another way, when each second electrode portion is in the retracted state, the signal line to the second electrode portion does not form a complete connection to the electrical shock system 12, whereas, when each second electrode portion is in the extended state, the signal line to the second electrode portion does form a complete connection to the electrical shock system 12. Accordingly, when each second electrode portion forms a part of the active electrode 250 (and thus in the extended state), the second electrode portion may receive the electrical shock from the electrical shock system 12.

To be specific, in the first defibrillation mode 210, only the first electrode portion 110 forms a complete (electrical) connection to the electrical shock system 12. Thus, only the first electrode portion 110 (which forms the active electrode 250) may receive the electrical shock. Meanwhile, in the second defibrillation mode 220, the first electrode portion 110 and the first second electrode portion 120a each form a (separate) complete connection to the electrical shock system 12. Thus, the first electrode portion 110 and the first second electrode portion 120a (which form the active electrode 250) may each receive the electrical shock. Finally, in the third defibrillation mode 230, the first electrode portion 110, the first second electrode portion 120a, and the second second electrode portion 120b each form a (separate) complete connection to the electrical shock system 12. Thus, the first electrode portion 110, the first second electrode portion 120a, and the second second electrode portion 120b (which form the active electrode 250) may each receive the electrical shock.

According to the second approach 302, the defibrillator paddle 100 comprises second electrical circuitry 320 different to the first electrical circuitry 310. Here, the second electrical circuitry 320 provides an electrical connection between the first electrode portion 110 and the electrical shock system 12. Additionally, the second electrical circuitry 320 allows for an electrical connection to form between different electrode portions. Particularly, each electrode portion comprises an electrical contact point (each electrical contact point forming part of the second electrical circuitry 320) which may (e.g., dependent on a positional arrangement) come into contact with the electrical contact point of another electrode portion, thus allowing an electrical shock provided to one electrode portion to be transferred to the other.

In the present example, only the first electrode portion 110 forms a dedicated electrical connection to the electrical shock system 12. Thus, in order to (also) receive the electrical shock, any second electrode portion must form an electrical connection with the first electrode portion 110. This may be a direct electrical connection (e.g., through direct contact with the electrical contact point of the first electrode portion 110) or an indirect electrical connection (e.g., via one or more other second electrode portions that form an electrical connection with the first electrode portion 110).

In some examples, each second electrode portion only forms an electrical connection with the first electrode portion 110 when the second electrode portion forms a part of the active electrode 250. Thus, each second electrode portion may only receive the electrical shock when they are actively being used for defibrillation.

To be specific, in the first defibrillation mode 210, neither of the first second electrode portion 120a and the second second electrode portion 120b form an electrical connection to the first electrode portion 110. Thus, only the first electrode portion 110 (which forms the active electrode 250) may receive the electrical shock from the electrical shock system 12. Meanwhile, in the second defibrillation mode 220, the first second electrode portion 120a (directly) forms an electrical connection to the first electrode portion 110. Thus, the first electrode portion 110 and the first second electrode portion 120a (which form the active electrode 250) may each receive the electrical shock from the electrical shock system 12. Finally, in the third defibrillation mode 230, both the first second electrode portion 120a and the second second electrode portion 120b form an electrical connection to the first electrode portions (the second second electrode portion 120b forming said connection via the first second electrode portion 120a). Thus, the first electrode portion 110, the first second electrode portion 120a, and the second second electrode portion 120b (which form the active electrode 250) may each receive the electrical shock from the electrical shock system 12.

As another approach, an electrical connection of each electrode portion to the electrical shock system 12 may be controlled by a respective switch. Accordingly, each switch may be opened or closed dependent on whether a corresponding electrode portion forms part of the active electrode 250. Said opening and closing of each switch may be controlled by a processing system of the defibrillator paddle 100. Particularly, the processing system may be (pre-) configured to determine which electrode portions are forming the active electrode responsive to the defibrillation mode of the defibrillator paddle 100 (or an indicator thereof) and control each switch accordingly.

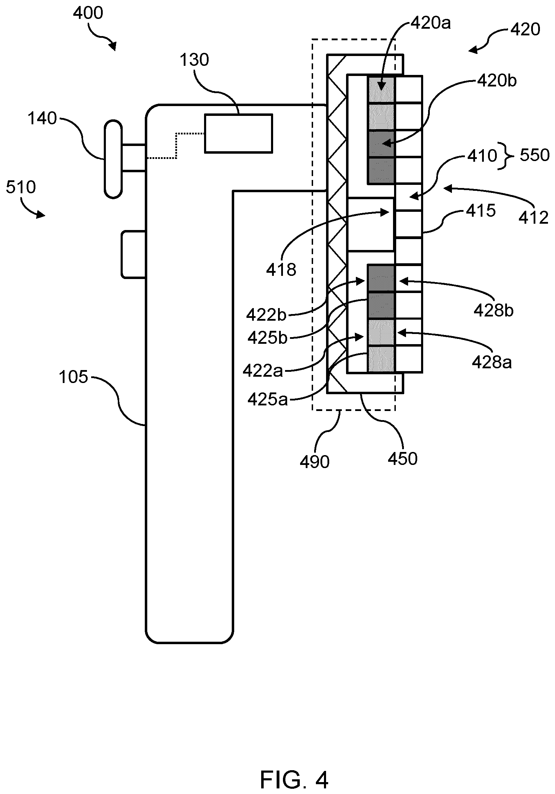

Fig. 4 illustrates another proposed defibrillator paddle 400 according to an embodiment of the invention. The defibrillator paddle 400 differs from the defibrillator paddle 100 of Fig. 1 in terms of its electrode portions. Specifically, the electrode portions of defibrillator paddle 400 are each separated into a number of segments. This enables more sophisticated movement of the one or more second electrode portions (relative to the first electrode portion). In particular, each segment (of a same second electrode portion) may move independent of another segment, e.g., perform a respective movement different to a movement of another segment, and/or become separated from another segment. This allows each second electrode portion to perform movements that would not be (physically) possible if it consisted of a single connected/continuous segment.

It will be appreciated that, in some examples, each second electrode portion segment may itself be considered as a second electrode portion.

The defibrillator paddle 400 comprises a first electrode portion 410 and one or more second electrode portions 420, in particular a first second electrode portion 420a and a second second electrode portion 420b. Each electrode portion comprises a shock surface, being the surface of the electrode portion intended to come into contact with the subject when said electrode portion is used for defibrillation. Particularly, the segments of each electrode portion each comprise a shock surface segment arranged on a side/surface of the segment, which together form the shock surface for the electrode portion.

It will be appreciated that the shock surface for an electrode portion may only be formed (i.e., exist) when the segments of the electrode portion are in certain arrangements. Particularly, each arrangement may correspond to a predetermined arrangement (of the segments of the electrode portion) for a defibrillation mode of the defibrillator paddle 400, e.g., being an arrangement in which the electrode portion may be used for defibrillating a subject.

The shock surface for each electrode portion is arranged at a first side of the electrode portion defining a front of the electrode portion. Additionally, a second side, opposite the first side (and thus opposite the shock surface), of each electrode portion defines a back of the electrode portion.

To be precise, in the present example, the first electrode portion 410 comprises a first shock surface 415 arranged on a first side 412 (of the first electrode portion 410) opposite a second side 418, the first second electrode portion 420a comprises a first second shock surface 425a arranged on a first side 422a (of the first second electrode portion 420a) opposite a second side 428a, and the second second electrode portion 420b comprises a second second shock surface 425b arranged on a first side 422b (of the second second electrode portion 420b) opposite a second side 428b.

The one or more second electrode portions 420 are configured to be moveable with respect to the first electrode portion 410, said movement being driven by the transmission mechanism 130. More particularly, each of the one or more second electrode portions 420 (or more precisely segments thereof) is/are configured to translate, rotate, expand, contract, and/or fold relative to the first electrode portion 410 to enable switching of the defibrillator paddle 400 between different defibrillation modes.

In Fig. 4, the defibrillator paddle 400 is depicted in a first defibrillation mode 510. In this defibrillation mode, each of the one or more second electrode portions 420 is arranged behind the first electrode portion 410, i.e., arranged at the second side 418 of the first electrode portion 410. Accordingly, each of the one or more second electrode portions 420 (or a shock surface thereof) is prevented or restricted from coming into contact with a subject due to being blocked by the first electrode portion 410. In other words, only the first electrode portion 410 may be used for defibrillating a subject. The first electrode portion 410 thus forms an active electrode 550 of the defibrillator paddle 400 when in the first defibrillation mode 510.

For use in later disclosure, in the first defibrillation mode 510, the second side of each second electrode portion is in contact with the second side 418 of the first electrode portion 410. The shock surface of each second electrode portion thus faces an opposite direction to the first shock surface 415.

The defibrillator paddle 400 may operate in the first defibrillation mode 510 responsive to the user-controlled switch 140 being in (or switched to) a first switching state. Particularly, in response to said switching state, the transmission mechanism 130 may drive movement of the one or more second electrode portions 420 so that they are arranged behind the first electrode portion 410, thus providing the respective positional arrangement associated with the first defibrillation mode 510.

A region 490 may be defined behind the active electrode 550. More specifically, the region 490 (or a side/surface thereof) faces the second side of each electrode portion forming the active electrode 550. For instance, in the first defibrillation mode 510, the region 490 faces the second side 418 of the first electrode portion 410. Accordingly, in the first defibrillation mode 510, each second electrode portion is arranged within the region 490.

More generally, in each defibrillation mode of the defibrillator paddle 400, any second electrode portion not forming the active electrode 550 (for the defibrillation mode) may be arranged within the region 490. In this way, each second electrode portion not forming the active electrode 550 is positioned behind the active electrode 550, thus preventing (or at least reducing a risk of) said second electrode portions from coming into contact with a subject. Particularly, when the active electrode 550 is brought into contact with the subject, the active electrode 550 may form a physical barrier between the subject and each second electrode portion not forming the active electrode 550, or at least create a gap/space between the subject and said second electrode portions, such that said second electrode portions are not in contact with the subject. Additionally, arranging each second electrode portion not forming the active electrode 550 in this way also prevents (or at least reduces a risk of) said second electrode portions blocking or hindering the active electrode 550 from coming into contact with the subject.

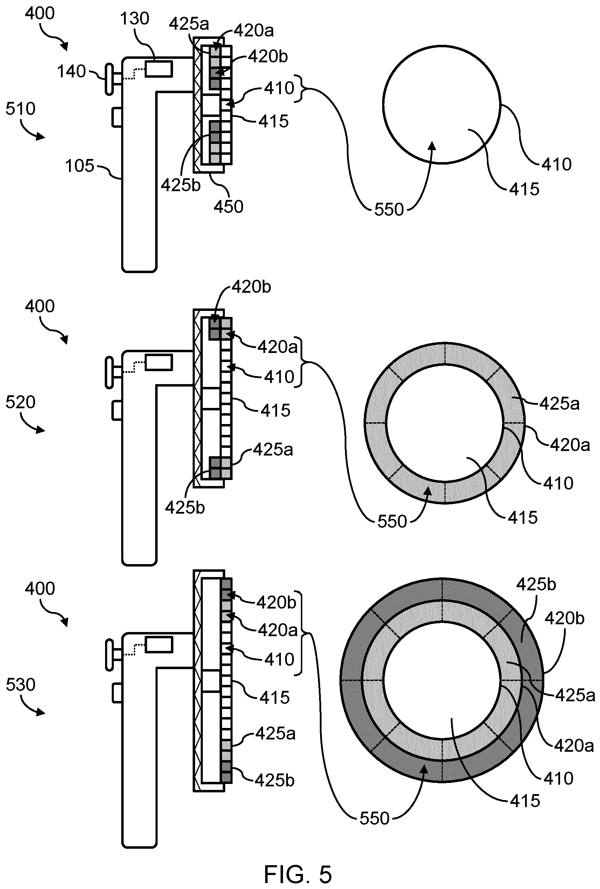

Fig. 5 illustrates further defibrillation modes of the defibrillator paddle 400, i.e., a second defibrillation mode 520 and a third defibrillation mode 530. Each defibrillation mode is associated with a different (i.e., respective) positional arrangement of the first second electrode portion 420a and the second second electrode portion 420b relative to the first electrode portion 410. Furthermore, each defibrillation mode is associated with a respective active electrode 550 formed from a respective combination of the first electrode portion 410, the first second electrode portion 420a and the second second electrode portion 420b.

On switching from the first defibrillation mode 510 to the second defibrillation mode 520, the first second electrode portion 420a moves such that it is aligned with the first electrode portion 410. More specifically, each segment of the first second electrode portion 420a is driven (by the transmission mechanism 130) to translate and rotate such that the resulting first second shock surface 425a faces a same direction and lies in a same (predefined) plane as the first shock surface 415. Accordingly, the first electrode portion 410 and the first second electrode portion 420a together form the active electrode 550 of the defibrillator paddle 400.

The defibrillator paddle 400 may be switched to the second defibrillation mode 520 responsive to the user-controlled switch 140 being switched to a second switching state. Particularly, in response to said switching state, the transmission mechanism 130 may drive movement of the first second electrode portion 420a to become aligned with the first electrode portion 410 (e.g., if it wasn't already), thus providing the respective positional arrangement associated with the second defibrillation mode 520.

On switching from the second defibrillation mode 520 to the third defibrillation mode 530, the second second electrode portion 420b moves such that it is aligned with the first electrode portion 410 and the first second electrode portion 420a. More specifically, each segment of the second second electrode portion 420b is driven (by the transmission mechanism 130) to translate and rotate such that the resulting second second shock surface 425b faces a same direction and lies in a same (predefined) plane as the first shock surface 415 and the first second shock surface 425a. Accordingly, the first electrode portion 410, the first second electrode portion 420a, and the second second electrode portion 420b together form the active electrode 550 of the defibrillator paddle 400.

The defibrillator paddle 400 may be switched to the third defibrillation mode 520 responsive to the user-controlled switch 140 being switched to a third switching state. Particularly, in response to said switching state, the transmission mechanism 130 may drive movement of the second second electrode portion 420b (and the first second electrode portion 420a) to become aligned with the first electrode portion 410 (e.g., if it/they weren't already), thus providing the respective positional arrangement associated with the third defibrillation mode 530.

In some examples, the defibrillation mode of the defibrillator paddle 400 may only be switched according to a predefined defibrillation mode sequence. Specifically, the defibrillator paddle 400 may only be switched to the first defibrillation mode 510 or the third defibrillation mode 530 from the second defibrillation mode 520. In other words, the defibrillator paddle 400 may not switch directly from the first defibrillation mode 510 to the third defibrillation 530 (and vice versa) but instead must switch via the second defibrillation mode 520 (thus following said predefined defibrillation mode sequence). This may be due to a connectedness of segments of the electrode portions. In particular, adjacent segments may be hingedly connected to each other such that they are able to rotate/hinge around (and translate with) a point on an adjacent segment. Consequently, the second second electrode portion 420b may be prevented or restricted from rotating to align with the first electrode portion 410 without also rotating the first second electrode portion 420a (thus requiring the defibrillator paddle 400 to switch to the second defibrillation mode 520 before the third defibrillation mode 530).

In response to the user-controlled switch 140 being switched directly from the first switching state to the third switching state (and vice versa), the transmission mechanism 130 may first switch the defibrillator paddle 400 to the second defibrillation mode 520 before switching the defibrillator paddle 400 to the third defibrillation mode 530 (or first defibrillation mode 510). Said switching from the first defibrillation mode 510 to the third defibrillation mode 530 (or vice versa) via the second defibrillation mode 520 may comprise a continuous switching movement/transition. In other examples, the user-controlled switch 140 may be prevented from switching directly between the first switching state and the third switching state.

An electrical connection between each electrode portion (or segments thereof) of the defibrillator paddle 400 and an electrical shock system (e.g., the electrical shock system of Fig. 1) may be controlled so that an electrical shock from the electrical shock system is only provided to some (i.e., not all) electrode portions, e.g., dependent on the defibrillation mode of the defibrillator paddle 400. In particular, said electrical connection(s) may be controlled so that only electrode portions forming the active electrode 550 of the defibrillator paddle 400 receive the electrical shock.

According to a first approach, each electrode portion (or segments thereof) may be connected to a respective signal line for (electrically) connecting the electrode portion to the electrical shock system. Each signal line may only form a complete connection to the electrical shock system when the corresponding electrode portion forms part of the active electrode 550. Particularly, movement of each electrode portion into a position where the electrode portion does not form part of the active electrode 550 (e.g., behind the active electrode 550) may break the connection between the electrode portion and the electrical shock system. Thus, each electrode portion may only receive the electrical shock when forming the active electrode 550. In some examples of the first approach, each electrode portion is electrically isolated from any other electrode portion and may only receive the electrical shock via a respective signal line.

According to a second approach, each electrode portion (or segments thereof) is/are permitted to form an electrical connection with other adjacent electrode portions/segments when forming the active electrode 550. Accordingly, an electrical shock provided to one electrode portion/segment maybe transferred to an adjacent electrode portion/segment. Specifically, according to the second approach, the electrical shock received by the defibrillator paddle 400 is provided to the first electrode portion 410. The electrical shock may then be provided to any second electrode portion/segment that forms an electrical connection with the first electrode portion 410, e.g., directly, or indirectly via one or more other electrode portions/segments.

In some examples of the second approach, only second electrode portions forming the active electrode 550 may electrically connect to the first electrode portion 410 (and thus only receive the electrical shock when forming the active electrode 550). To achieve this, certain parts of the electrode portions may be coated/covered in a, insulating or non-conductive layer. For example, the second side of each electrode portion may be covered in a non-conductive support layer. Accordingly, electrode portions arranged at the second side of the active electrode 550 (e.g., second electrode portions not forming part of the active electrode 550) will be electrically isolated from the active electrode 550. An electrical shock provided to the active electrode 550 may therefore be prevented or restricted from being transferred to electrode portions not forming part of the active electrode 550.

In some examples, the defibrillator paddle 400 further comprises an insulating cover 450 arranged within the region 490. The insulating cover 450 is arranged such that each second electrode portion not forming the active electrode 550 is arranged between the insulating cover 450 and the active electrode 550.

The insulating cover 450 differs from the insulating cover 150 of Fig. 1 in that the insulting cover 450 is configured to expand and contract dependent on the defibrillation mode of the defibrillator paddle 400. Put another way, the bounds of the insulating cover 450 are configured to change (i.e., expand or contract) responsive to the defibrillation mode of the defibrillator paddle 400. This is depicted in Fig. 5, where the insulating cover 450 is shown to scale dependent on the (lateral) size of the active electrode 550. Accordingly, in each defibrillation mode, a projection of the active electrode 550 for the defibrillation mode onto the insulating cover 450 is entirely contained within bounds of the insulating cover 450.

In this way, the insulating cover 450 (or a lateral extent thereof) is more similarly sized to the active electrode 550 in each defibrillation mode. Thus, in each defibrillation mode, an amount of redundant area of the insulating cover 450 is reduced. This makes the defibrillator paddle 400 easier to handle/maneuver in certain defibrillation modes due to the reduced physical extent/size of the insulating cover 450.

To facilitate expansion and contraction, the insulating cover 450 may comprise a malleable material capable of stretching and relaxing (e.g., an elastic material), and/or a folded or segmented structure capable of expanding (e.g., unfolding) and contracting/compressing (e.g., folding).

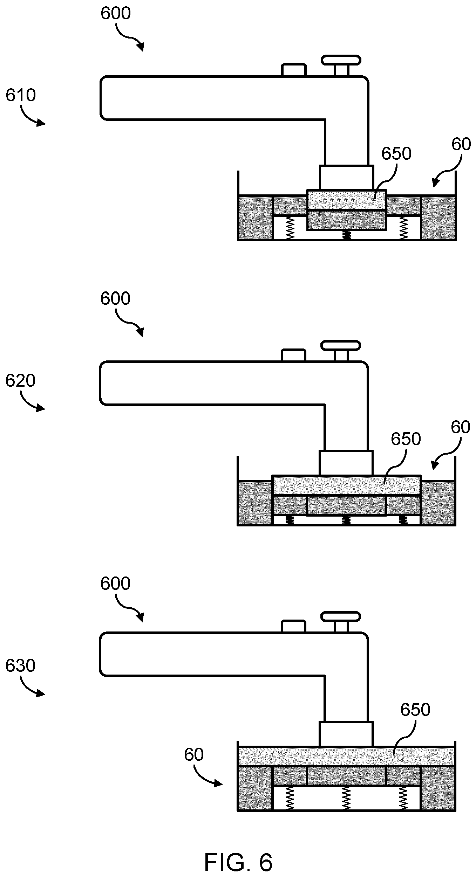

Fig. 6 illustrates another proposed defibrillator paddle 600 arranged within a paddle tray 60. The defibrillator paddle 600 comprises an active electrode 650 which is received by the paddle tray 60. The defibrillator paddle is configured to operate in three, different defibrillation modes 610, 620, 630, each associated with a differently sized active electrode 650.

The paddle tray 60 is configured for storage of the defibrillator paddle 600, e.g., when the defibrillator paddle 600 is not in use. In particular, the paddle tray 60 is configured to receive (the active electrode 650 of) the defibrillator paddle 600 to safely store the defibrillator paddle 600 (e.g., reduce a risk of electrocution from the active electrode 650), and protect the active electrode 650 from damage/wear.

The paddle tray 60 is further configured to receive the defibrillator paddle 600 in any defibrillation mode of the defibrillator paddle 600. Particularly, the paddle tray 60 is configured to receive the active electrode 650 regardless of the defibrillation mode (and thus regardless of the size of the active electrode 650). To achieve this, the paddle tray comprises a plurality of adaptable/adjustable sections for receiving active electrodes of different size.

In the present example, the paddle tray comprises a plurality of spring-loaded sections whose dimensions match those of the active electrode 650 in particular defibrillation modes. Accordingly, when the dimensions of the active electrode 650 match those of one or more of the plurality of spring-loaded sections, the active electrode 650 compresses said spring-loaded sections, creating a recess for the active electrode 650 to be received within.

The paddle tray 60 may form part of the defibrillator 10 of Fig. 1. In examples where the defibrillator 10 comprises more than one (proposed) defibrillator paddle (e.g., two defibrillator paddles) the defibrillator 10 may comprise a respective paddle tray 60 for each defibrillator paddle.

In accordance with the present disclosure, each electrode portion of any herein proposed defibrillator paddle may comprise a flexible material. This facilities elastic deformation (e.g., bending, twisting, stretching, etc.) of (at least a part of) each electrode portion, reducing strain/stress in each electrode portion from applied forces. In particular, this provides increased maneuverability of each second electrode portion, allowing them to be more easily moved relative to the first electrode portion.

In some examples, the flexible material faces outwardly from each electrode portion, thus allowing (at least a part of) the surface of each electrode portion to be deformed. Particularly, the flexible material may be arranged on (at least) the first side of each electrode portion. This allows each electrode portion to more easily shape to a part of a subject's body (e.g., when pressed against the subject) improving contact between the electrode portion and the subject.

In some examples, the flexible material has a negative Poisson's ratio. This enhances the strength and damage (e.g., fracture, indentation) resistance of the flexible part of the electrode portion.

When each electrode portion comprises a flexible material, the second side of the electrode portion (i.e., the side opposite the shock surface of the electrode portion) may be covered in a (rigid) support layer, e.g., a non-conductive support layer. This provides increased structural support (i.e., rigidness) to the electrode portion, while still allowing for some flexibility of the electrode portion.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A defibrillator paddle comprising:
a plurality of electrode portions, each electrode portion comprising a first side; a second side, opposite to the first side; and a shock surface on the first side of the electrode portion, wherein the plurality of electrode portions comprises:
a first electrode portion; and
one or more second electrode portions configured to be moveable with respect to the first electrode portion; and
a transmission mechanism configured to drive movement of the one or more second electrode portions relative to the first electrode portion, wherein:
the defibrillator paddle is switchable between at least two defibrillation modes for defibrillation of a subject, wherein each of the at least two defibrillation modes is associated with:
a respective positional arrangement of the one or more second electrode portions relative to the first electrode portion; and
a respective subset of the first electrode portion and the one or more second electrode portions forming an active electrode of the defibrillator paddle for defibrillation of the subject, wherein the respective subset comprises either i) only the first electrode portion or ii) the first electrode portion and at least one of the one or more second electrode portions; and
the transmission mechanism is configured to switch the defibrillator paddle between the at least two defibrillation modes by driving movement of at least one of the one or more second electrode portions.

2. The defibrillator paddle of claim 1, wherein, in each of the at least two defibrillation modes of the defibrillator paddle, the shock surface of each electrode portion forming the active electrode for the defibrillation mode lies in a predefined plane for said defibrillation mode.

3. The defibrillator paddle of any one of claims 1 or 2, wherein:
each active electrode for the at least two defibrillation modes of the defibrillator paddle is associated with a shock surface area being the sum of the area of the shock surface of each electrode portion forming the active electrode; and
each active electrode for the at least two defibrillation modes of the defibrillator paddle is associated with a different shock surface area.

4. The defibrillator paddle of any one of claim 1 to 3, wherein, in each of the at least two defibrillation modes of the defibrillator paddle, each electrode portion forming the active electrode for the defibrillation mode is in physical contact with at least one other electrode portion forming said active electrode.

5. The defibrillator paddle of any one of claims 1 to 4, wherein each electrode portion comprises a flexible material.

6. The defibrillator paddle of any one of claims 1 to 5, wherein the second side of each electrode portion is covered in a non-conductive support layer.

7. The defibrillator paddle of any one of claims 1 to 6, wherein, in each of the at least two defibrillation modes of the defibrillator paddle, any second electrode portion not forming the active electrode for the defibrillation mode is arranged in a region facing the second side of each electrode portion forming said active electrode.

8. The defibrillator paddle of any one of claims 1 to 7, wherein:
the defibrillator paddle is configured to receive an electrical shock from an electrical shock system; and
in each of the at least two defibrillation modes of the defibrillator paddle, the electrical shock is provided to at least each electrode portion forming the active electrode for the defibrillation mode.

9. The defibrillator paddle of claim 8, wherein:
the electrical shock is provided to the first electrode portion; and
in each of the at least two defibrillation modes of the defibrillator paddle, each second electrode portion forming the active electrode for the defibrillation mode is configured to form an electrical connection with the first electrode portion.

10. The defibrillator paddle of any one of claims 1 to 9, wherein, the defibrillator paddle further comprises an insulating cover arranged in a region facing the second side of each electrode portion forming the active electrode for any defibrillation mode of the defibrillator paddle, wherein, in each of the at least two defibrillation modes of the defibrillator paddle, a projection of the active electrode for the defibrillation mode onto the insulating cover is entirely contained within bounds of the insulating cover.

11. The defibrillator paddle of claim 10, wherein the bounds of the insulating cover are configured to change responsive to the defibrillation mode of the defibrillator paddle.

12. The defibrillator paddle of any one of claims 1 to 11, wherein:
the defibrillator paddle further comprises a user-controlled switch switchable between at least two switching states, each switching state being associated with a respective defibrillation mode of the at least two defibrillation modes of the defibrillator paddle; and
the transmission mechanism is configured to switch the defibrillator paddle to any one of the at least two defibrillation modes in response to the user-controlled switch being in the switching state associated with said defibrillation mode.

13. A defibrillator comprising:
an electrical shock system;
a processing system configured to control the electrical shock system; and
at least one defibrillator paddle according to any one of claims 1 to 12, wherein the at least one defibrillator paddle is configured to receive an electrical shock from the electrical shock system.

14. The defibrillator of claim 13, wherein the processing system is configured to control at least an energy of any electrical shock provided by the electrical shock system to the at least one defibrillator paddle responsive to an indication of the at least one defibrillator paddle operating in any one of the at least two defibrillation modes.

15. The defibrillator of any one of claims 13 or 14, further comprising, for each defibrillator paddle, a paddle tray configured to receive the defibrillator paddle in any defibrillation mode of the defibrillator paddle.
